# EUROPEAN PATENT SPECIFICATION

(11) **EP 1 617 914 B1**
(45) Date of publication and mention of the grant of the patent: **15.10.2008**
(21) Application number: 04728223.1
(22) Date of filing: 19.04.2004
(51) Int. Cl.: A61Q 15/00, A61Q 13/00, A61K 8/06, A61L 9/00

(54) **VOC-FREE MICROEMULSIONS**
MIKROEMULSIONEN OHNE FLÜCHTIGE ORGANISCHE VERBINDUNGEN
MICROEMULSIONS EXEMPTES DE COMPOSES ORGANIQUES VOLATILS

(30) Priority: 21.04.2003 US 421216
(43) Date of publication of application: 25.01.2006
(73) Proprietor: FIRMENICH SA, 1211 Genève 8 (CH)
(72) Inventor: VLAD, Florin, Joseph, Annandale, NJ 08801 (US)
(74) Representative: Salvaterra-Garcia, Maria de Lurdes
(86) International application number: PCT/IB2004/001473
(87) International publication number: WO 2004/093836

(56) References cited:
- EP-A- 0 334 777
- EP-A- 1 243 185
- WO-A-01/85121
- US-A- 5 252 555
- US-A- 5 468 725

## Description

### Technical Field

The present invention relates to the field of perfumery and more precisely it provides a perfume or a perfuming composition in the form of a highly transparent water-based VOC-free microemulsion.

Other aspects of the invention are the use of said microemulsion as perfuming ingredient and a consumer article associated with said microemulsion.

### Background Art

Generally speaking, water-based microemulsions containing a perfume have been already reported in the prior art. Frequently, said microemulsions, in order to the dispersion of the perfume, which is an oil, contain large amounts of short chain alcohols or other VOC compounds. The problem of said microemulsions is that VOC are nowadays considered as undesired for various reasons.

In general terms, VOC-free microemulsions that may be useful for the perfumery industry have already been described in the prior art. Generally, in formulating such microemulsions is it important to increase the total amount of surfactants so as to obviate the absence of VOCs, otherwise the final emulsions display a lack of clarity and/or stability problems, and this is not acceptable in perfumery. However, the increase of the amount of surfactants in the final microemulsions results in a product containing a surfactant system that is in large excess with respect of the perfume. Obviously, a large excess of surfactant is also a disadvantage for the final product.

The US5468725 describes an alcohol-free transparent perfume comprising water and a stable transparent oil-in-water microemulsion flavour concentrate formed of water, at least one hydrophobic perfume oil, at least one cationic surfactant and at least one non-ionic surfactant in the absence of lower alkanols. It does not refer to a solubilizing-aid ingredient.

The WO01/85121 refers to a clear emulsion antiperspirant and/or deodorant cosmetic composition comprising a water-in-oil emulsion which is free of simple glycols and low and middle chain alcohols though it incorporates polyethylene glycols and inorganic or organic salts, amino acids or urea. The composition has a NTU of 50 or lower. It does not refer to a o/w transparent microemulsion.

Therefore, there is still a need for a perfume, or perfuming product, in the form of a microemulsion that is free of VOCs, and is able to associate a priced crystal-clear appearance, and optionally also a prolonged stability, with a content in surfactants which does not exceed the perfume content.

### Description of the invention

We have now surprisingly discovered that the use of a suitable amount of an appropriate solubilizing-aid ingredient, in addition to the classical ingredients, namely a perfume, a surfactant system and water, can solve the problem cited above.

Therefore, a first object of the present invention is a perfume or perfuming composition in the form of VOC-free oil-in-water (o/w) , transparent microemulsion comprising:
A) from 1 to 30% w/w of an oil having a surface tension, at 25°C, of at least 25 mN/m;
B) a surfactant system, comprising one ore more ionic surfactants, such as anionic, cationic and/or amphoteric surfactants, in a quantity of at least 50% w/w of the surfactant system, and one or more non-ionic surfactant, in a quantity of at most 50% w/w of the surfactant system; the w/w oil/surfactant system ratio being comprised between 1 and 3;
C) from 0.1 to 10% w/w of a solubilizing-aid ingredient selected from the group consisting of the ammonium, alkaline and alkaline earth salts of:
   i) a C₅-C₁₀ compound comprising an aromatic or non aromatic five or six member heterocyclic ring and one or two carboxylic functional groups ;
   ii) a C₂-C₇ linear, branched or cyclic mono-, di- or tri-carboxylic acid ;
   iii) benzoic, hydroxyl-benzoic or amino-benzoic acid, a C₈-C₁₂ benzoic, hydroxyl-benzoic or amino-benzoic acid substituted by one or two C₁-C₅ alkyl groups ;
   iv) benzene-sulfonic acid, a C₇-C₁₁ benzene-sulfonic acid substituted by one or two C₁-C₅ alkyl groups, optionally hydroxylated naphthalene-sulfonic acid, an optionally hydroxylated C₁₁-C₁₆ naphthalene-sulfonic acid substituted by one or two C₁-C₅ alkyl groups;
   v) a halide, ascorbate, bicarbonate, thiocyanate; or
   vi) a mixture thereof; and
D) at least 60% of water_{;}
said microemulsion having a clarity comprised between 0 and 90 NTU, when measured between 400 and 600 nm in a 2.5 cm cell at 25°C.

The abbreviation w/w represents the weight to weight ratio, as the ratio between the weight of a specific ingredient and the weight of the microemulsion.

By "VOC" we mean here the Volatile Organic Compounds as defined by the Environmental Protection Agency, and in particular we mean C₁-C₅ alkanols, such as ethanol, or C₁-C₅ alkandiols, such as ethylene glycol.

By "microemulsion" we mean here a dispersion that forms spontaneously and has a droplet size comprised between 10 and 150 nm, at a temperature comprised between 0° and 80°C. However, according to a particular embodiment of the invention, the present microemulsion has a droplet size comprised between 10 and 60 nm, or even between 10 and 40 nm, at a temperature comprised between 0° and 80°C.

By "clarity" we mean here the measure of the light scattered, at an angle of 90°, by the invention's microemulsion. According to a praised embodiment of the invention, the microemulsion has a clarity comprised between 0 and 50 NTU when measured in the same conditions as specified above.

In a particular embodiment of the invention, a specific range of surface tension can also characterizes the invention's microemulsion. Indeed, according to a particular embodiment of the invention the microemulsions have a surface tension, measured at 25°C, comprised between 22 and 30mN/m, or even comprised between 24 and 28mN/m. Therefore, said microemulsions can contain large amounts of low polar oils.

The possibility to have crystal clear VOC-free microemulsion containing large amounts of low polar oils was another unexpected result. Indeed, it is known to a person skilled in the art that it is particularly difficult to dissolve low polar oils in water, especially in large amounts, without the use of some VOCs or oil/surfactant ratio below 1.

As mentioned above, the invention's microemulsions can also display a very good stability, e.g. phase separation is not observed within a reasonable frame of time. Indeed, the invention's microemulsions are commonly stable for at least 30 days, at temperatures comprised between 2° and 60°C. Furthermore, in some cases nearly thermodynamic stability, e.g. more than 6 months at temperatures comprised between 2° and 45°C, was achieved.

However, it has to be mentioned that the range of temperatures in which the invention's microemulsion shows very good stability is a function of the amount, as well as the exact nature, of the oil, surfactant system and solubilizing-aid ingredient used. Therefore in some cases it is possible that the stability temperature range of the named microemulsions may be narrower, e.g. from 5° to 45°C only, or wider, e.g. from 0° to 80°C.

By "oil" we mean here a lipophylic organic liquid that is essentially insoluble in water. An example of suitable oil is a liquid that comprises at least 75% w/w, or even at least 90% w/w, of a perfume or a perfuming composition. Said oil may also consist of a perfume. Moreover, another example of suitable oil is a flavor or flavoring composition.

In particular, as the perfume or perfuming composition there can be used any perfuming ingredient or, as happens more often, any mixture of perfuming ingredients currently used in perfumery, e.g. of compounds capable of imparting an hedonic olfactive effect to the composition in which they are added. Said perfuming ingredients can be of natural or synthetic origin. Although a detailed description of said perfuming ingredients would be in any case not exhaustive, in a general manner it can be mentioned that these ingredients belong to chemical classes as varied as alcohols, aldehydes, ketones, esters, ethers, acetates, nitriles, terpene hydrocarbons, nitrogenous or sulphurous heterocyclic compounds and essential oils of natural or synthetic origin. The nature of these ingredients can be found in specialized books of perfumery, e.g. in S. Arctander (Perfume and Flavor Chemicals, Montclair N.J., USA 1969) or similar textbooks of reference, and a more detailed description thereof is not warranted here. A skilled person being able to select said ingredients on the basis of his general knowledge and according to the nature of the product to be perfumed and the desired olfactory effect.

The oil, especially when comprising a perfume, may optionally also contains a suitable solvent, in a quantity of up to 25 % w/w of the oil, but preferably less than 10% w/w. The presence of a solvent, which is not a VOC, may be useful to have a monophasic oil or to modulate surface tension of said oil. As example of suitable solvents, one may cite polar or non-polar low molecular weight solvent such as isoparaffins, paraffins, hydrocarbons silicon oils, perfluorinated aliphatic ethers, glycol ethers, glycol ether esters, esters, or ketones. Non-restrictive examples of such solvents includes dimethicone or cyclomethicone, which are commercialized by Chemsil Silicon INC. under the trade names Cosmetic Fluid^{®} 1288, and respectively Cosmetic Fluid^{®} 1387, jojoba oil, perfluoroisobutyl methyl ether, diethyl phthalate, dipropylene glycol and isopropyl myristate.

Other possible ingredients of the oil are fixatives.

Concerning the physical properties of the oil, we have already mentioned that the latter must have a surface tension comprised in a specific range. According to a particular embodiment, the oil will have a surface tension comprised between 25 and 40 mN/m, at 25°C. Therefore, it is possible to use as oil a low polar oil, in particular a low polar perfume. By "low polar oil or perfume" we mean here, for example, an oil or perfume rich in highly hydrophobic ingredients or an oil or perfume that contains only small amounts of polar solvents or completely free of polar solvents.

As low polar perfumes one can mention those containing from 5% w/w, or even 20% w/w, to 99% w/w of terpenes or/and from 5 to 30% w/w of musks; percentages being relative to the weight of the oil.

Said terpenes may be of wood or citrus origin and example of which are terpineol. or d-limonene. A non-restrictive example of musks is hexadecanolide.

As mentioned above, the oil represents between 1 to 30% of the microemulsion total weight. According to a particular embodiment the oil content represents preferably from 3, or even 5, to 20% w/w, in respect to the microemulsion total weight.

The surfactant system, which is another mandatory element of the invention, may be described as consisting of a ionic fraction, representing more than 50% w/w of the surfactant system, and a non-ionic fraction, representing less than 50% w/w of the surfactant system.

The ionic fraction contains a single ionic surfactant or, according to an embodiment of the invention, a mixture of ionic surfactants. The ionic surfactants are of the anionic, cationic or amphoteric type.

Suitable anionic surfactants comprise the salts of C₆-C₂₄ mono- or di- sulfonic, alkylsulfuric, alkylarylsulfuric, alkylarylphosphate or carboxylic acids and also the polyethylene glycol co-polymers with sulfonic or carboxylic acids. Specific, but not limiting examples of said anionic surfactants are sodium, potassium, ammonium or mono-, di- or tri-ethanolammonium salts of C₆-C₁₂ dialkyl sulfosuccinic acids (such as sodium dioctyl-sulfosuccinate), C₇-C₂₄ alkarylsulfonic acids (such as sodium dodecyl benzenesulfonate), C₆-C₁₅ alkylsulfuric acid (such as sodium dodecylsulfate), C₁₀-C₂₀ acyl glutamic acid (such as sodium cocoyl glutamate), or polyethylene glycol/dimethicone sulfosuccinic acids (such as disodium PEG-12 dimethicone sulfosuccinate known under the trade name Mackanate^{®} DC-50 from The McIntyre Group).

Suitable cationic surfactants comprise the salts of C₁₀-C₃₅ ammonium derivatives of fatty acids, alcohols, alkylamidoalkylmorpholine or amines and also the IPDI (isophorone diisocyanate) co-polymers with said ammonium derivatives or with fatty amines and optionally polyethylene glycols. Specific, but not-limiting examples, of said cationic surfactants are halides, sulfates or carboxylates of C₂₀₋₃₀ quaternary ammonium alkyl (such as hexadecyltrimethyl ammonium bromide or didodecylammonium bromide), C₁₋₄ alkyl N-cocoyl-L-arginate (such as DL-2-pyrrolidone-5-carboxylic acid salt of ethyl N-cocoyl-L-arginate commercialized by Ajinomoto Co., Inc. under the trade name CAE^{®}), (C₁₀₋₂₀ amido) (C₁₋₄ alkyl) morpholine (such as isostearamidopropyl morpholine lactate), IPDI copolymers with N-C₁₀₋₂₀amido(C₁₋₄ alkyl)-N,N-di(C₁₋₄ alkyl)-N-C₁₋₄ alkyl) Ammonium (such as bis(N-Ricinolemidopropyl-N,N-Dimethyl)/N-Ethyl Ammonium Sulfate/IPDI Copolymer also known under the trademark Polyquat^{®} PPI-RC from ALZO) or polyethylene glycol/C₁₀-C₂₀ fatty alkyl amine/IPDI copolymers (such as the PEG Cocamine/IPDI Copolymeric surfactants also known under the trademark Polyderm^{®} PPI-CA-15 from ALZO).

Suitable amphoteric surfactants comprise C₁₀-C₂₅ betaines, amphoacetates and imidazoline derivatives, as well as the polyethylene glycol/fatty amine/glycine/IPDI copolymes. Specific, but not-limiting, examples of said amphoteric surfactants are the C₁₀-C₂₀ fatty amido C₂-C₅ alkyl betaines (such as cocoamidopropyl betaine), coco- and lauro-amphoacetates (such as sodium cocoamphoacetate known under the trade name Mackam^{®} HPC-32 commercialized by McIntyre Group), and the polyethylene glycol/C₁₀-C₂₀ fatty alkyl amine/glycine/IPDI copolymers (such as PEG-13 soyamine-Glycine/IPDI Copolymer also known under the trademark Polytaine^{®} PPI-SA-15 from ALZO).

The non-ionic fraction may contain a single non-ionic surfactant or, according to an embodiment of the invention, a mixture of non-ionic surfactants with an HLB value which comprises between 9 and 18. Suitable examples of said surfactants includes ethoxylated and/or propoxylated (C₅-C₁₂ alkyl)phenols ethers containing 5 to 20 EO or PO units (such as polyethylene glycol nonylphenyl ethers, polyethylene glycol octylphenyl ethers, also known under the generic tradename Polystep^{®}), polyethylene glycol sorbitol ether containing 3 to 30 EO units (such as sorbitol esters with oleic, myristic, stearic, palmitic acid also known as those known under the tradenames Tweens^{®} from ICI or Glycosperse^{®} from LONZA), sucrose esters with C₈-C₂₀ fatty acid (such as sucrose esters with oleic, palmitic or stearic acid, such as Ryoto Sugar Ester M-1695 commercialized by Mitsubishi-Kagaku Foods Corporation), ethoxylated aliphatic C₆-C₂₀ alcohols containing 2 to 30 EO units (such as ethoxylated secondary C₆-C₂₀ alcohols), C₈-C₂₀ polyglyceryl esters (such as glycerol-polyethylene glycol oxystearate commercialized by BASF under the trade name Chromophor^{®} CO40), polyethylene glycol and polypropylene glycol block copolymers (such as those known under the tradename Pluronics^{®} from BASF), ethoxylated glycol ether containing 2 to 30 EO units (such as PEG-10 stearyl ether also known under the trade name Volpo^{®} S-10 from CRODA), or polyethylene glycol mono- or-diester of aliphatic C₅-C₁₁ carboxylic acids containing 2 to 10 EO units (EO stands for ethylene oxide and PO stands for propylene oxide).

The amount of surfactant system needed to obtain a microemulsion according to the invention depends essentially on the amount of oil present and on the solubilizing-aid ingredient used. In a general manner, when the solubilizing-aid ingredient is used in amounts such as disclosed further below, we have noticed that the w/w oil/surfactant system ratio is comprised between 1 and 3. However, frequently it is possible to obtain a ratio comprised between 1.2, or even 1.5, and 2.0.

Another mandatory ingredient of the invention's microemulsions is the solubilizing-aid ingredient. By the expression "solubilizing-aid ingredient" we mean here an organic or inorganic salt, or a precursor thereof, of low molecular mass, e.g. below 400g/mol. As solubilizing-aid ingredient it can also be used a mixture of said salts.

Said compounds, which per their nature are neither surfactants nor solvents, have been found to improve the solubility of organic compounds in water. In fact, and unexpectedly, these salts, or their precursors, are able to enhance the oil-solubilization capacity of the surfactant system. In other words, in the presence of a solubilizing-aid ingredient, as defined above or below, the same amount of surfactants is able to solubilize in the water phase more oil than if the solubilizing-aid ingredient was not present.

Moreover, the clarity of the microemulsion thus obtained is significantly and unexpectedly improved by the presence of the solubilizing-aid ingredient.

Thus, the presence of at least one of the above-mentioned salts has been found to be essential in order to ensure an oil/surfactant system ratio of at least 1, as well as a crystal-clear appearance, i.e. a high clarity or, if preferred, a low turbidity.

According to a particular embodiment of the invention, suitable salts are selected from the group consisting of sodium, potassium, magnesium and calcium salts of pyridine carboxylic acids, proline acid, pyrrolidone carboxylic acid, benzoic acid, hydroxyl-benzoic acid, amino-benzoic acid, L-lactic acid, L-ascorbic acid, bicarbonate, halide, succinic acid, oxalic acid, tartaric acid, citric acid, a C₈-C₁₀ derivative of benzoic, hydroxyl-benzoic or amino-benzoic acid substituted by one or two C₁-C₃ alkyl groups (such as the sodium salt of p-methyl-benzoic acid or of p-isopropyl-hydroxyl-benzoic acid), benzene-sulfonic acid, a C₇-C₉ benzene-sulfonic acid substituted by one or two methyl or ethyl groups (such as potassium toluene sulfonate), optionally hydroxylated naphthalene-sulfonic acid, an optionally hydroxylated C₁₁-C₁₆ naphthalene-sulfonic acid substituted by one or two C₁-C₅ alkyl groups (such as sodium butylnaphtalene sulfonate), C₃ to C₆ alkanoic acids (such as the sodium salt of pentanoic acid), and any mixture of said salts.

According to a particular embodiment of the invention, the solubilizing-aid ingredient may be advantageously chosen between the following compounds: pyrrolidone carboxylic acid sodium salt (also known as Ajidew NL-50 from Ajinomoto), sodium benzoate, sodium L-lactate, calcium L-ascorbate, sodium bicarbonate or di-sodium succinate. Any mixtures of said salts can also be used.

As mentioned above, the solubilizing-aid ingredient is present in an amount comprised between 0.1 and 10% w/w in respect to the total weight of the microemulsion.

The exact amount solubilizing-aid need to obtain a microemulsion according to the invention depends on the exact nature of the oil, of the surfactant mixture, in particular of the w/w ratio of ionic and non-ionic fractions, and on the amount of oil present. However, in general and according to a particular embodiment, said amount is advantageously comprised between 0.1 and 5% w/w, or even between 0.1 and 2% w/w.

Concerning the fourth components of the present microemulsion, i.e. water, it is useful to mention that it is preferable to use de-ionised water.

The invention's microemulsion can also comprise, as optional components, one or more ingredients such as colorants, anti-microbial agents, antioxidants, preservatives, chelating agents or UV-inhibitors. Such types of materials are well known to a person skilled in the art and do not need a more detailed description. Whenever said ingredients are added to the microemulsion, then they will represent no more than 3 % w/w, or even 2% w/w, the percentages being relative to the total weight of the microemulsion.

The invention's microemulsion can be prepared according to any method known in the art. A suitable method consists in dissolving into the water the surfactant system, to form a clear micellar solution. To the resulting micellar solution are added under gentle stirring the solubilizing-aid ingredient, and whenever necessary the optional ingredients to form an initial oil-free microemulsion. Under gentle mixing the resulting oil-free microemulsion can easily solubilize the corresponding amount of perfume to form an isotropic clear, single-phase microemulsion product. High mechanical forces such as shear forces are not necessary to manufacture the present microemulsion.

The above-mentioned oil-free microemulsion is therefore an excellent solubilizing medium which allows to obtain highly clear microemulsions containing an oil such as perfumes or flavors. Therefore an oil-free microemulsion comprising
I) from 1.0 to 45% w/w of a surfactant system, as defined above;
ll) from 0.2 to 15% w/w of a solubilizing-aid ,as defined above; and
III) water up to 100%;
said oil-free microemulsion having a surface tension, at 25°C, comprised between 20 and 35 mN/m, is also another aspect of the present invention.

According to a preferred embodiment of the invention, in the oil-free microemulsion, the w/w amount of surfactant system ranges between 3.5% and 15%, while the w/w amount of solubilizing-aid ranges between 0.1% and 10%, or even between 0.1% and 5%.

As anticipated above, the microemulsion of the invention is particularly suitable for the manufacture of consumer articles capable of dispensing a perfume in the surrounding space. Said consumer articles are also an object of the present invention.

A suitable consumer article comprises a microemulsion as described above together with a suitable container and optionally a means to produce an aerosol. Nonlimiting examples of such consumer article are room deodorants, or air fresheners, as well as hair or skin preparations, such as fine perfumery articles.

In another embodiment, said consumer articles comprise a microemulsion according to the invention and a consumer product base. For the sake of clarity, it has to be mentioned that, by "consumer product base" we mean here a consumer product which is compatible with perfuming ingredients. In other words, a perfumed article according to the invention comprises the functional formulation, as well as optionally additional benefit agents, corresponding to a consumer product, e.g. a detergent or an air freshener, and an olfactive effective amount of at least one invention's microemulsion. A suitable consumer product base is, for examples, a surface cleaning product, an hygiene product, an hair care product such as shampoos, a body-care product, a cosmetic preparation, a fabric refreshers, an ironing water or a wipe.

The nature and type of the constituents of the consumer product do not warrant a more detailed description here, which in any case would not be exhaustive, the skilled person being able to select them on the basis of its general knowledge and according to the nature and the desired effect of said product.

A further object of the present invention is the use of a microemulsion according to the invention as a perfuming ingredient. In other words, a method to confer, enhance, improve or modify the odor properties of a composition or of an article, which method comprise adding to said composition or article an olfactive effective amount of a microemulsion as defined above.

The invention will now be described in further detail by way of the following examples.

### Example 1

### Preparation of perfume-in water microemulsion according to the invention

A perfume containing almost 70% of terpenes was obtained by ad-mixing the following ingredients:

| Ingredient | Parts by weight |
|---|---|
| Benzyl salicylate | 21.00 |
| Exaltolide^{® 1)} | 15.00 |
| Grapefruit oil | 200.00 |
| Iso E Super²⁾ | 21.00 |
| Lilial^{® 3)} | 20.00 |
| Mandarin oil sfuma | 55.00 |
| (Z)-3-Methyl-2-(2-pentenyl)-2-cyclopenten-1-one | 1.00 |
| Hedione^{® 4)} | 100.00 |
| Nerol | 10.00 |
| Orange oil | 150.00 |
| Spearmint oil | 2.00 |
| Styrallyl acetate | 5.00 |
| Total | 600.00 |

| | |
|---|---|
| 1) Pentadecanolide; origin: Firmenich SA, Geneva, Switzerland 2) 1-(octahydro-2,3,8,8-tetramethyl-2-naphtalenyl)-1-ethanone origin: International Flavors & Fragrances, USA 3) 3-(4-tert-butylphenyl)-2-methylpropanal; origin: Givaudan-Roure SA, Vernier, Switzerland 4) Methyl dihydrojasmonate; origin: Firmenich SA, Geneva, Switzerland | |

In a general way, a microemulsion according to the invention was obtained, in a first step, by mixing together in a beaker the de-ionized water, the surfactants, and the solubilizing-aid ingredient. The mixture is gently stirred at room temperature for a few minutes by means of any common mixing device, such as a magnetic stirring. Optionally, during the stirring the mixture may be protected by a nitrogen blanket. In the second step, the fragrance is added under stirring over the above water solution. The microemulsion was formed in few minutes. Usually a clear product was obtained in less than 10 minutes, sometimes even instantly.
Following this general procedure, it was obtained a microemulsion according to Table 1 and 2, and having an oil/surfactant system ratio of 1.71:

**Table 1: formulation of the microemulsion**

| Ingredient | Parts by weight | |
|---|---|---|
| Perfume | 15.15 | |
| Surfactant system: | 8.87 | |
| *PEO (20) Sorbitan monooleate* | | *2.07* |
| *PEO (20) Sorbitan monolaurate* | | *0.65* |
| *Solubilisant LRI* ¹⁾ | | 1.63 |
| *Sodium Dioctyl Sulfosuccinate* | | 3.87 |
| *Sodium Dodecylsulfate* | | 0.65 |
| Solubilizing-aid ingredient : | 1.13 | |
| *Sodium Benzoate* | | *0.40* |
| *Ajidew NL-50* ²⁾ | | *0.40* |
| *Sodium L-lactate* | | *0.33* |
| Optional ingredient: | 0.35 | |
| *Glydant Plus* ³⁾ | | *0.35* |
| De-ionised water | 74.50 | |
| Total | 100.00 | |

| | | |
|---|---|---|
| 1) 89.7% Aqueous Blend of non-ionic surfactants from LCW 2) Sodium Pirrolydone Carboxylic Acid 50% aqueous solution from Ajinimoto Inc. 3) 87% Aqueous solution, origin: Lonza | | |

**Table 2: physical properties of the microemulsion described in Table 1**

| | |
|---|---|
| Clarity ¹⁾ | |
| T = 25°C | 75.1 NTU (oil droplet size: 24.5 nm) |
| T = 0-2°C | 76.9 NTU (oil droplet size: 26.7 mn) |
| Surface tension (mN/m) | 26.44 ± 0.05 |
| Temperature Stability | from 0°C to 60°C |
| Viscosity (25° C) ²⁾ | 7.02 cPs |

| | |
|---|---|
| 1) NTU is Nephelometric Turbidity Unit, measured on a Turbidimeter VWR Model 66120-200, with a tungsten lamp and 2 photo voltaic cells centered at 90° to the incident light, response between 400 and 600nm. 2) Measured with aBrookfield Viscometer with spindle YULA-15 at 30 rpm | |

### Example 2

### Preparation of perfume-in water microemulsion according to the invention

Following the same procedure as described in Example 1, it was obtained a microemulsion according to Table 3 and 4, and having an oil/surfactant system ratio of 1.09:

**Table 3: formulation of the microemulsion**

| Ingredient | Parts by weight | |
|---|---|---|
| Perfume ¹⁾ | 10.16 | |
| Surfactant system: | 9.34 | |
| *PEO (20) Sorbitan monooleate* | | *2.17* |
| *PEO (20) Sorbitan monolaurate* | | *0. 68* |
| *Solubilisant LRI* ¹⁾ | | *1.72* |
| *Sodium Dioctyl Sulfosuccinate* | | *4. 09* |
| *Sodium Dodecylsulfate* | | *0. 68* |
| Solubilizing-aid ingredient : | 1.36 | |
| *Ajidew NL-50* ¹⁾ | | *1.36* |
| De-ionised water | 79.14 | |
| Total | 100.00 | |

| | | |
|---|---|---|
| 1) as in Example 1 | | |

**Table 4: physical properties of the microemulsion described in table 3**

| | |
|---|---|
| Clarity ¹⁾ | |
| T = 25°C | 27.6 NTU (oil droplet size: 14.5 nm) |
| T = 0-2°C | 30.3 NTU (oil droplet size: 15.9 nm) |
| Surface tension (mN/m) | 26.58 ± 0.04 |
| Temperature Stability | from 0°C to 65°C |
| Viscosity (25° C)²⁾ | 4.14 cPs |

| | |
|---|---|
| 1) as in Example 1 | |

### Example 3

### Preparation of perfume-in water microemulsion according to the invention

A perfume was obtained by admixing the following ingredients:

| Ingredients | Parts by weight |
|---|---|
| Citronellyl acetate | 3 |
| Geranyl acetate . | 9 |
| Linalyl acetate | 276 |
| 10% * Aldehyde C10 | 3 |
| 10% * Aldehyde C12 | 12 |
| Methyl anthranilate | 16 |
| Bergamote essential oil | 226 |
| Cetalox^{® 1)} | 5 |
| Lemon essential oil | 318 |
| Dihydromyrcenol ²⁾ | 60 |
| Dipropylene glycol | 20 |
| 10% * Elemi ³⁾ | 20 |
| Fleuria 41063 B ⁴⁾ | 3 |
| Ethyl linalol | 66 |
| 10% * 3-(4-Methoxyphenyl)-2-methylpropanal ⁴⁾ | 30 |
| Geraniol | 6 |
| 50% * Habanolide^{® 5)} | 130 |
| Hedione^{® 6)} | 215 |
| Hedione^{®} HC ⁷⁾ | 72 |
| 10% ** Indol | 12 |
| Iso E super⁸⁾ | 85 |
| Lavandin grosso essential oil | 26 |
| 1% * Liffarome^{® 9)} | 20 |
| Linalol | 40 |
| Mandarine sfuma essential oil | 5 |
| 10% * Spearmint essential oil | 30 |
| Neroli bigarade essential oil | 130 |
| Orange essential oil | 80 |
| Phenethylol | 9 |
| Petitgrain essential oil | 63 |
| Pipol | 5 |
| Rosemary essential oil | 16 |
| Terpineol | 9 |
| Violet essential oil | 50 |
| 1% * Zestover ¹⁰⁾ | 30 |
| Total | 2100 |

| | |
|---|---|
| * in dipropylene glycol DIPG) ** in triethanolamine 1) 8,12-epoxy-13,14,15,16-tetranorlabdane 2) origin : International Flavors and Fragrances, USA 3) 5-Allyl-1,2,3-trimethoxybenzene; origin: Calchauvet, Grasse, France 4) origin : Firmenich SA, Geneva, Switzerland 5) pentadecenolide; origin : Firmenich SA, Geneva, Switzerland 6) Methyl dihydrojasmonate ; origin : Firmenich SA, Geneva, Switzerland 7) Methyl dihydrojasmonate with a high content of cis isomer; origin : Firmenich SA, Geneva, Switzerland 8) 1-(octahydro-2,3,8,8-tetrarmethyl-2-naphtalenyl)-1-ethanone; origin: International Flavors and Fragrances, USA 9) 3-Hexenyl methyl carbonate; origin: International Flavors and Fragrances, USA 10) 9-decen-1-ol; origin: International Flavors and Fragrances, USA | |

Following the same procedure as described in Example 1, and the perfume above described, it was obtained a microemulsion according to Table 5 and 6, and having a oil/surfactant system ratio of 1.71:

**Table 5: formulation of the microemulsion**

| Ingredient | Parts by weight | |
|---|---|---|
| Perfume | 15.18 | |
| Surfactant system: | 8.89 | |
| *PEO (20) Sorbitan monooleate* | | *2.08* |
| *PEO (20) Sorbitan monolaurate* | | *0.64* |
| *Solubilisant LRI* ¹⁾ | | 1.64 |
| *Sodium Dioctyl Sulfosuccinate* | | 3.89 |
| *Sodium Dodecylsulfate* | | *0.64* |
| Solubilizing-aid ingredient: | 0.98 | |
| *Sodium Benzoate* | | *0.40* |
| *Ajidew NL-50* ¹⁾ | | *0.33* |
| *Sodium L-lactate* | | 0.25 |
| Optional ingredient: | 0.37 | |
| *Glydant Plus* ³⁾ | | *0.37* |
| De-ionised water | 74.58 | |
| Total | 100.00 | |

| | | |
|---|---|---|
| 1) as in Example 1 | | |

**Table 6: physical properties of the microemulsion described in table 5**

| | |
|---|---|
| Clarity ¹⁾ | |
| T = 25°C | 48.4 NTU (oil droplet size: 28.3 nm) |
| T = 0-2°C | 49.5 NTU (oil droplet size: 29.8 nm) |
| Surface tension (mN/m) | 26.44 ± 0.05 |
| Temperature Stability | from 0°C to 57°C |
| Viscosity (25° C) ²⁾ | 7.98 cPs |

| | |
|---|---|
| 1) as in Example 1 | |

### Example 4

### Preparation of perfume-in water microemulsion according to the invention

Following the same procedure as described in Example 1, it was obtained a microemulsion according to Table 7 and 8, and having a oil/surfactant system ratio of 1.09:

**Table 7: formulation of the microemulsion**

| Ingredient | Parts by weight | |
|---|---|---|
| Perfume ²⁾ | 10.31 | |
| Surfactant system: | 9.46 | |
| *PEO (20) Sorbitan monooleate* | | *2.20* |
| *PEO (20) Sorbitan monolaurate* | | *0. 69* |
| *Solubilisant LRI* ¹⁾ | | *1.75* |
| *Sodium Dioctyl Sulfosuccinate* | | 4.13 |
| *Sodium Dodecylsulfate* | | *0. 69* |
| Solubilizing-aid ingredient : | 0.67 | |
| *Sodium Benzoate* | | *0.40* |
| *Ajidew NL-50* ¹⁾ | | *0.27* |
| Optional ingredient: | 0.34 | |
| *Glydant Plus* ¹⁾ | | *0.34* |
| De-ionised water | 79.22 | |
| Total | 100.00 | |

| | | |
|---|---|---|
| 1) as in Example 1 2) as in example 3 | | |

**Table 8: physical properties of the microemulsion described in table 3**

| | |
|---|---|
| Clarity ¹⁾ | |
| T = 25°C | 14.0 NTU (oil droplet size: 15.9 nm) |
| T = 0-2°C | 14.2 NTU (oil droplet size: 18,0 nm) |
| Surface tension (mN/m) | 26.66 ± 0.05 |
| Temperature Stability | from 0°C to 70°C |
| Viscosity (25° C) ²⁾ | 4.50 cPs |

| | |
|---|---|
| 1) as in Example 1 | |

## Claims

1. A perfume or perfuming composition in the form of a VOC-free o/w transparent microemulsion comprising:
A) from 1 to 30% w/w of an oil having a surface tension, at 25°C, of at least 25 mN/m;
B) a surfactant system, comprising one ore more ionic surfactants, in a quantity of at least 50% w/w of the surfactant system, and one or more non-ionic surfactant, in a quantity of at most 50% w/w of the surfactant system; the w/w oil/surfactant system ratio being comprised between 1 and 3;
C) from 0.1 to 10% w/w of a solubilizing-aid ingredient selected from the group consisting of the ammonium, alkaline and alkaline earth salts of:
i) a C₅-C₁₀ compound comprising an aromatic or non aromatic five or six member heterocyclic ring and one or two carboxylic functional groups ;
ii) a C₂-C₇ linear, branched or cyclic mono-, di- or tri-carboxylic acid ;
iii) benzoic, hydroxyl-benzoic or amino-benzoic acid, a C₈-C₁₂ benzoic, hydroxyl-benzoic or amino-benzoic acid substituted by one or two C₁-C₅ alkyl groups ;
iv) benzene-sulfonic acid, a C₇-C₁₁ benzene-sulfonic acid substituted by one or two C₁-C₅ alkyl groups, optionally hydroxylated naphthalene-sulfonic acid, an optionally hydroxylated C₁₁-C₁₆ naphthalene-sulfonic acid substituted by one or two C₁-C₅ alkyl groups;
v) a halide, ascorbate, bicarbonate, thiocyanate; or
vi) a mixture thereof; and
D) at least 60% of water;
said microemulsion having a clarity comprised between 0 and 90 NTU, when measured between 400 and 600 nm in a 2.5 cm cell at 25°C.

2. A composition according to claim 1, wherein the clarity is comprised between 0 and 50 NTU, when measured between 400 and 600 nm in a 2.5 cm cell at 25°C.

3. A composition according to claim 1 or 2, wherein said microemulsion has a surface tension, measured at 25°C, comprised between 22 and 30mN/m..

4. A composition according to any one of claims 1 to 3, wherein said oil comprises at least 75% of a perfume.

5. A composition according to claim 4, wherein said oil comprises at least 90% of a perfume.

6. A composition according to claim 4, wherein said perfume contains from 5% w/w to 99% w/w of terpenes or/and from 5 to 30% w/w of musks.

7. A composition according to any one of claims 1 to 6, wherein the oil has a surface tension comprised between 25 and 40 mN/m, at 25°C.

8. A composition according to any one of claims 1 to 7, wherein the w/w oil/surfactant system ratio is comprised between 1.2 and 2.0.

9. A composition according to any one of claims 1 to 8, wherein
a) the anionic surfactants are selected from the group consisting of sodium, potassium, ammonium and mono-, di- and tri-ethanolammonium salts of C₆-C₁₂ dialkyl sulfosuccinic acids, C₇-C₂₄ alkarylsulfonic acids, C₆-C₅ alkylsulfuric acid, C₁₀-C₂₀ acyl glutamic acid, and polyethylene glycol/dimethicone sulfosuccinic acids;
b) the cationic surfactants are selected from the group consisting of halides, sulfates or carboxylates of C₂₀₋₃₀ quaternary ammonium alkyl, C₁₋₄ alkyl N-cocoyl-L-arginate, (C₁₀₋₂₀ amido) (C₁₋₄ alkyl) morpholine, IPDI copolymers with N-C₁₀₋₂₀amido(C₁₋₄ alkyl)-N,N-di(C₁₋₄ alkyl)-N-(C₁₋₄ alkyl) Ammonium, and polyethylene glycol/C₁₀-C₂₀ fatty alkyl amine/IPDI copolymers;
c) the amphoteric surfactants are selected from the group consisting of C₁₀-C₂₀ fatty amido C₂-C₅ alkyl betaines, coco- and lauro-amphoacetates and the polyethylene glycol/C₁₀-C₂₀ fatty alkyl amine/glycine/IPDI copolymers;
d) the non-ionic surfactants are selected from the group consisting of ethoxylated and propoxylated (C₅-C₁₂ alkyl)phenols ethers containing 5 to 20 EO or PO units, polyethylene glycol sorbitol ether containing 3 to 30 EO units, sucrose esters with C₆-C₂₀ fatty acid, ethoxylated aliphatic C₆-C₂₀ alcohols containing 2 to 30 EO units, C₈-C₂₀ polyglyceryl esters, polyethylene glycol and polypropylene glycol block copolymers, ethoxylated glycol ether containing 2 to 30 EO units, and polyethylene glycol mono- and -diester of aliphatic C₅-C₁₁ carboxylic acids containing 2 to 10 EO units.

10. A composition according to any one of claims 1 to 9, wherein the solubilizing-aid ingredient is selected from the group consisting of sodium, potassium, magnesium and calcium salts of pyridine carboxylic acids, proline acid, pyrrolidone carboxylic acid, benzoic acid, hydroxyl-benzoic acid, amino-benzoic acid, L-lactic acid, L-ascorbic acid, bicarbonate, halide, succinic acid, oxalic acid, tartaric acid, citric acid, a C₈-C₁₀ derivative of benzoic, hydroxyl-benzoic or amino-benzoic acid substituted by one or two C₁-C₃ alkyl groups, benzene-sulfonic acid, a C₇-C₉ benzene-sulfonic acid substituted by one or two methyl or ethyl groups, naphthalene-sulfonic acid, an optionally hydroxylated C₁₁-C₁₄ naphthalene-sulfonic acid substituted by one or two C₁-C₄ alkyl groups, a C₃ to C₆ alkanoic acid and any mixture of said salts.

11. A composition according to claim 10, wherein the solubilizing-aid ingredient is selected from the group consisting of pyrrolidone carboxylic acid sodium salt, sodium benzoate, sodium L-lactate, calcium L-ascorbate, sodium bicarbonate, di-sodium succinate and any mixture of said salts.

12. A composition according to any one of claims 1 to 11, wherein the solubilizing-aid ingredient represents from 0.1 to 5% w/w of the microemulsion.

13. A composition according to any one of claims 1 to 12, wherein said microemulsion has a surface tension, at 25°C, comprised between 22 and 30mN/m.

14. A consumer article comprising a composition, as defined in any one of claims 1 to 13, together with a container and optionally a means to produce an aerosol.

15. A consumer article according to claim 14, in the form of a room deodorant or a hair or skin preparation.

16. A consumer article comprising a composition, as defined in any one of claims 1 to 13, and a consumer article base.

17. A method to confer, enhance, improve or modify the odor properties of a product or of an articles, which method comprises adding to said product or article ant olfactive effective amount of a composition as defined in claim 1.

18. An oil-free and VOC-free solubilizing medium comprising:
I) from 1.0 to 45% w/w of a surfactant system, as defined in claim 1;
II) from 0.2 to 15% w/w of a solubilizing-aid, as defined in claim 1; and
III) water up to 100%;
said oil-free medium having a surface tension, at 25°C, comprised between 20 and 35 mN/m.

19. A solubilizing medium according to claim 18, comprising from 3.5 to 15% w/w of a surfactant system, as defined in claim 1, and from 0.1 to 10% w/w of a solubilizing-aid, as defined in claim 1.

20. A solubilizing medium according to claim 18, wherein the solubilizing-aid ingredient is selected from the group consisting of pyrrolidone carboxylic acid sodium salt, sodium benzoate, sodium L-lactate, calcium L-ascorbate, sodium bicarbonate, disodium succinate and any mixture of said salts.

21. A process for the preparation of a composition according to claim 1, wherein there is added to a solubilizing medium according to claim 18 an appropriate amount of oil, preferably a fragrance oil, to form a transparent microemulsion.

## Patentansprüche

1. Parfüm oder parfümierende Zusammensetzung in der Form einer VOC-freien transparenten Ö/W-Mikroemulsion, umfassend:
A) von 1 bis 30% Gew./Gew. eines Öls mit einer Oberflächenspannung bei 25°C von mindestens 25 mN/m;
B) ein Tensidsystem, umfassend ein oder mehrere ionische Tenside in einer Menge von mindestens 50% Gew./Gew. des Tensidsystems, und ein oder mehrere nichtionische Tenside in einer Menge von höchstens 50% Gew./Gew. des Tensidsystems; wobei das Gew./Gew.-Öl/Tensidsystem-Verhältnis zwischen 1 und 3 liegt;
C) von 0,1 bis 10% Gew./Gew. eines solubilisierenden Hilfsbestandteils, ausgewählt aus der Gruppe, bestehend aus den Ammonium-, Alkali- und Erdalkalisalzen von:
i) einer C₅-C₁₀-Verbindung, umfassend einen aromatischen oder nichtaromatischen fünf- oder sechsgliedrigen heterocyclischen Ring und ein oder zwei carboxylische funktionelle Gruppen;
ii) einer linearen, verzweigten oder cyclischen C₂-C₇-Mono-, -Di- oder -Tricarbonsäure;
iii) Benzoe-, Hydroxylbenzoe- oder Aminobenzoesäure, einer C₈-C₁₂-Benzoe-, -Hydroxylbenzoe- oder -Aminobenzoesäure, substituiert durch eine oder zwei C₁-C₅-Alkylgruppen;
iv) Benzolsulfonsäure, einer C₇-C₁₁-Benzolsulfonsäure, substituiert durch eine oder zwei C₁-C₅-Alkylgruppen, gegebenenfalls hydroxylierter Naphthalinsulfonsäure, einer gegebenenfalls hydroxylierten C₁₁-C₁₆-Naphthalinsulfonsäure, substituiert durch eine oder zwei C₁-C₅-Alkylgruppen;
v) einem Halogenid, Ascorbat, Bicarbonat, Thiocyanat; oder
vi) einem Gemisch davon; und
D) mindestens 60% Wasser;
wobei die Mikroemulsion eine Klarheit zwischen 0 und 90 NTU hat, wenn zwischen 400 und 600 nm in einer 2,5-cm-Zelle bei 25°C gemessen wird.

2. Zusammensetzung nach Anspruch 1, wobei die Klarheit zwischen 0 und 50 NTU liegt, wenn zwischen 400 und 600 nm in einer 2,5-cm-Zelle bei 25°C gemessen wird.

3. Zusammensetzung nach Anspruch 1 oder 2, wobei die Mikroemulsion eine Oberflächenspannung, gemessen bei 25°C, zwischen 22 und 30 mN/m hat.

4. Zusammensetzung nach einem der Ansprüche 1 bis 3, wobei das Öl mindestens 75% eines Parfüms umfaßt.

5. Zusammensetzung nach Anspruch 4, wobei das Öl mindestens 90% eines Parfüms umfaßt.

6. Zusammensetzung nach Anspruch 4, wobei das Parfüm von 5% Gew./Gew. bis 99% Gew./Gew. Terpene oder/und von 5 bis 30% Gew./Gew. Moschusstoffe enthält.

7. Zusammensetzung nach einem der Ansprüche 1 bis 6, wobei das Öl eine Oberflächenspannung zwischen 25 und 40 mN/m bei 25°C hat.

8. Zusammensetzung nach einem der Ansprüche 1 bis 7, wobei das Gew./Gew.-Öl/Tensidsystem-Verhältnis zwischen 1,2 und 2,0 liegt.

9. Zusammensetzung nach einem der Ansprüche 1 bis 8, wobei
a) die anionischen Tenside aus der Gruppe, bestehend aus Natrium-, Kalium-, Ammonium- und Mono-, Di- und Triethanolammoniumsalzen von C₆-C₁₂-Dialkylsulfosuccinsäuren, C₇-C₂₄-Alkarylsulfonsäuren, C₆-C₁₅-Alkylschwefelsäure, C₁₀-C₂₀-Acylglutaminsäure und Polyethylenglycol/Dimethicon-Sulfosuccinsäuren, ausgewählt sind;
b) die kationischen Tenside aus der Gruppe, bestehend aus Halogeniden, Sulfaten oder Carboxylaten von quaternärem C₂₀₋₃₀-Ammoniumalkyl, C₁₋₄-Alkyl-N-cocoyl-L-arginat, (C₁₀₋₂₀-Amido)-(C₁₋₄-alkyl)-morpholin, IPDI-Copolymeren mit N-C₁₀₋₂₀-Amido-(C₁₋₄-alkyl)-N,N-di(C₁₋₄-alkyl)-N-(C₁₋₄-alkyl)ammonium und Polyethylenglycol/C₁₀-C₂₀-Fettalkylamin/IPDI-Copolymeren, ausgewählt sind;
c) die amphoteren Tenside aus der Gruppe, bestehend aus C₁₀-C₂₀-Fettamido-C₂-C₅-alkylbetainen, Coco- und Lauroamphoacetaten und den Polyethylenglycol/C₁₀-C₂₀-Fettalkylamin/Glycin/IPDI-Copolymeren, ausgewählt sind;
d) die nichtionischen Tenside aus der Gruppe, bestehend aus ethoxylierten und propoxylierten (C₅-C₁₂-Alkyl)phenolethern, enthaltend 5 bis 20 EO- oder PO-Einheiten, Polyethylenglycolsorbitolether, enthaltend 3 bis 30 EO-Einheiten, Saccharoseestern mit C₈-C₂₀-Fettsäure, ethoxylierten aliphatischen C₆-C₂₀-Alkoholen, enthaltend 2 bis 30 EO-Einheiten, C₈-C₂₀-Polyglycerylestern, Polyethylenglycol- und Polypropylenglycol-Blockcopolymeren, ethoxyliertem Glycolether, enthaltend 2 bis 30 EO-Einheiten, und Polyethylenglycolmono- und -diester von aliphatischen C₅-C₁₁-Carbonsäuren, enthaltend 2 bis 10 EO-Einheiten, ausgewählt sind.

10. Zusammensetzung nach einem der Ansprüche 1 bis 9, wobei der solubilisierende Hilfsbestandteil aus der Gruppe, bestehend aus Natrium-, Kalium-, Magnesium- und Calciumsalzen von Pyridincarbonsäuren, Prolinsäure, Pyrrolidoncarbonsäure, Benzoesäure, Hydroxylbenzoesäure, Aminobenzoesäure, L-Milchsäure, L-Ascorbinsäure, Bicarbonat, Halogenid, Succinsäure, Oxalsäure, Weinsäure, Citronensäure, einem C₈-C₁₀-Derivat von Benzoe-, Hydroxylbenzoe- oder Aminobenzoesäure, substituiert durch eine oder zwei C₁-C₃-Alkylgruppen, Benzolsulfonsäure, einer C₇-C₉-Benzolsulfonsäure, substituiert durch eine oder zwei Methyl- oder Ethylgruppen, Naphthalinsulfonsäure, einer gegebenenfalls hydroxylierten C₁₁-C₁₄-Naphthalinsulfonsäure, substituiert durch eine oder zwei C₁-C₄-Alkylgruppen, einer C₃- bis C₆-Alkansäure und einem Gemisch der Salze, ausgewählt ist.

11. Zusammensetzung nach Anspruch 10, wobei der solubilisierende Hilfsbestandteil aus der Gruppe, bestehend aus Pyrrolidoncarbonsäure-Natriumsalz, Natriumbenzoat, Natrium-L-lactat, Calcium-L-ascorbat, Natriumbicarbonat, Dinatriumsuccinat und einem Gemisch dieser Salze, ausgewählt ist.

12. Zusammensetzung nach einem der Ansprüche 1 bis 11, wobei der solubilisierende Hilfsbestandteil von 0,1 bis 5% Gew./Gew. der Mikroemulsion darstellt.

13. Zusammensetzung nach einem der Ansprüche 1 bis 12, wobei die Mikroemulsion eine Oberflächenspannung bei 25°C zwischen 22 und 30 mN/m hat.

14. Verbrauchergegenstand, umfassend eine Zusammensetzung, wie definiert in einem der Ansprüche 1 bis 13, zusammen mit einem Behälter und gegebenenfalls einem Mittel zum Erzeugen eines Aerosols.

15. Verbrauchergegenstand nach Anspruch 14 in der Form einer Raumdeodorants oder einer Haar- oder Hautzubereitung.

16. Verbrauchergegenstand, umfassend eine Zusammensetzung, wie definiert in einem der Ansprüche 1 bis 13, und eine Verbrauchergegenstandbasis.

17. Verfahren zum Übertragen, Verstärken, Verbessern oder Modifizieren der Geruchseigenschaften eines Produkts oder eines Gegenstands, welches Verfahren Hinzufügen einer olfaktorisch wirksamen Menge einer Zusammensetzung, wie definiert in Anspruch 1, zu dem Produkt oder Gegenstand umfaßt.

18. Öl-freies und VOC-freies solubilisierendes Medium, umfassend:
I) von 1,0 bis 45% Gew./Gew. eines Tensidsystems, wie definiert in Anspruch 1;
II) von 0,2 bis 15% Gew./Gew. eines solubilisierenden Hilfsstoffes, wie definiert in Anspruch 1; und
III) Wasser bis zu 100%;
wobei das Öl-freie Medium eine Oberflächenspannung bei 25°C zwischen 20 und 35 mN/m hat.

19. Solubilisierendes Medium nach Anspruch 18, umfassend von 3,5 bis 15% Gew./Gew. eines Tensidsystems, wie definiert in Anspruch 1, und von 0,1 bis 10% Gew./Gew. eines solubilisierenden Hilfsstoffes, wie definiert in Anspruch 1.

20. Solubilisierendes Medium nach Anspruch 18, wobei der solubilisierende Hilfsbestandteil aus der Gruppe, bestehend aus Pyrrolidoncarbonsäure-Natriumsalz, Natriumbenzoat, Natrium-L-lactat, Calcium-L-ascorbat, Natriumbicarbonat, Dinatriumsuccinat und irgendeinem Gemisch dieser Salze, ausgewählt ist.

21. Verfahren für die Zubereitung einer Zusammensetzung nach Anspruch 1, wobei zu einem solubilisierenden Medium nach Anspruch 18 eine geeignete Menge von Öl, vorzugsweise einem Duftöl, hinzugegeben wird, um eine transparente Mikroemulsion zu erzeugen.

## Revendications

1. Parfum ou composition parfumante sous la forme d'une microémulsion transparente huile/eau (o/w) exempte de composés organiques volatils (COV) comprenant:
A) 1 à 30% p/p d'une huile ayant une tension superficielle, à 25°C, d'au moins 25 mN/m;
B) un système tensioactif, comprenant un ou plusieurs tensioactifs ioniques, en une quantité d'au moins 50% p/p du système tensioactif, et un ou plusieurs tensioactifs non ioniques, en une quantité tout au plus de 50% p/p du système tensioactif; le rapport huile/système tensioactif p/p étant compris entre 1 et 3;
C) 0,1 à 10% p/p d'un ingrédient facilitant la solubilisation choisi dans le groupe constitué des sels d'ammonium et des sels alcalins et alcalino-terreux de:
i) un composé en C₅-C₁₀ comprenant un noyau hétérocyclique à cinq ou six atomes de carbone, aromatique ou non aromatique, et un ou deux groupes fonctionnels carboxyliques;
ii) un acide mono-, di- ou tri-carboxylique linéaire, ramifié ou cyclique, en C₂-C₇;
iii) l'acide benzoïque, hydroxy-benzoïque ou amino-benzoïque, un acide benzoïque, hydroxy-benzoïque ou amino-benzoïque en C₈-C₁₂ substitué par un ou deux groupes alkyle en C₁-C₅;
iv) l'acide benzènesulfonique, un acide benzènesulfonique en C₇-C₁₁ substitué par un ou deux groupes alkyle en C₁-C₅, un acide naphtalènesulfonique éventuellement hydroxylé, un acide naphtalènesulfonique en C₁₁-C₁₆ éventuellement hydroxylé substitué par un ou deux groupes alkyle en C₁-C₅;
v) un halogénure, ascorbate, bicarbonate, thiocyanate; ou
vi) un mélange des précédents; et
D) au moins 60% d'eau;
ladite microémulsion ayant une turbidité comprise entre 0 et 90 NTU, lorsqu'on la mesure entre 400 et 600 nm dans une cellule de 2,5 cm à 25°C.

2. Composition selon la revendication 1, dans laquelle la turbidité est comprise entre 0 et 50 NTU, lorsqu'on la mesure entre 400 et 600 nm dans une cellule de 2,5 cm à 25°C.

3. Composition selon la revendication 1 ou 2, dans laquelle ladite microémulsion a une tension superficielle, mesurée à 25°C, comprise entre 22 et 30 mN/m.

4. Composition selon l'une quelconque des revendications 1 à 3, dans laquelle ladite huile comprend au moins 75% d'un parfum.

5. Composition selon la revendication 4, dans laquelle ladite huile comprend au moins 90% d'un parfum.

6. Composition selon la revendication 4, dans laquelle ledit parfum contient 5% p/p à 99% p/p de terpènes et/ou 5 à 30% p/p de muscs.

7. Composition selon l'une quelconque des revendications 1 à 6, dans laquelle l'huile a une tension superficielle comprise entre 25 et 40 mN/m, à 25°C.

8. Composition selon l'une quelconque des revendications 1 à 7, dans laquelle le rapport huile/système tensioactif p/p est compris entre 1,2 et 2,0.

9. Composition selon l'une quelconque des revendications 1 à 8, dans laquelle
a) les tensioactifs anioniques sont choisis dans le groupe constitué des sels de sodium, potassium, ammonium et mono-, di- et tri-éthanolammonium d'acides (dialkyl en C₆-C₁₂)sulfo-succiniques, d'acides (alkaryl en C₇-C₂₄)sulfoniques, d'acides (alkyl en C₆-C₁₅)sulfuriques, d'acides (acyl en C₁₀-C₂₀)-glutamiques, et d'acides polyéthylèneglycol/diméthicone-sulfosucciniques;
b) les tensioactifs cationiques sont choisis dans le groupe constitué des halogénures, sulfates ou carboxylates d'alkylammonium quaternaire en C₂₀-₃₀, du N-cocoyl-L-arginate d'alkyle en C₁₋₄, de la (amido en C₁₀-₂₀)(alkyl en C₁₋₄)morpholine, des copolymères d'IPDI avec le N-(amido en C₁₀₋₂₀)(alkyl en C₁₋₄)-N,N-di(alkyl en C₁₋₄)-N-(alkyl en C₁₋₄)ammonium, et des copolymères de polyéthylèneglycol/alkyl-amine d'acide gras en C₁₀-C₂₀/IPDI;
c) les tensioactifs amphotères sont choisis dans le groupe constitué des (amido d'acide gras en C₁₀-C₂₀)(alkyl en C₂-C₅)bétaïnes, des coco- et lauro-amphoacétates, et des copolymères de polyéthylèneglycol/alkylamine d'acide gras en C₁₀-C₂₀/glycine/IPDI;
d) les tensioactifs non ioniques sont choisis dans le groupe constitué des éthers d'(alkyl en C₅-C₁₂)phénols éthoxylés et propoxylés contenant 5 à 20 unités OE ou OP, d'un éther de sorbitol de polyéthylèneglycol contenant 3 à 30 unités OE, des esters de saccharose avec un acide gras en C₈-C₂₀, des alcools en C₆-C₂₀ aliphatiques éthoxylés contenant 2 à 30 unités OE, des esters polyglycéryliques en C₈-C₂₀, des copolymères séquencés de polyéthylèneglycol et de polypropylèneglycol, d'un éther de glycol éthoxylé contenant 2 à 30 unités OE, et des mono- et di-esters de polyéthylèneglycol d'acides carboxyliques en C₅-C₁₁ aliphatiques contenant 2 à 10 unités OE.

10. Composition selon l'une quelconque des revendications 1 à 9, dans laquelle l'ingrédient facilitant la solubilisation est choisi dans le groupe constitué des sels de sodium, potassium, magnésium et calcium des acides pyridine-carboxyliques, de l'acide proline, de l'acide pyrrolidone-carboxylique, de l'acide benzoïque, de l'acide hydroxybenzoïque, de l'acide aminobenzoïque, de l'acide L-lactique, de l'acide L-ascorbique, d'un bicarbonate, d'un halogénure, de l'acide succinique, de l'acide oxalique, de l'acide tartrique, de l'acide citrique, d'un dérivé en C₈-C₁₀ de l'acide benzoïque, hydroxybenzoïque ou aminobenzoïque substitué par un ou deux groupes alkyle en C₁-C₃, de l'acide benzènesulfonique, d'un acide benzènesulfonique en C₇-C₉ substitué par ou un deux groupes méthyle ou éthyle, de l'acide naphtalènesulfonique, d'un acide naphtalènesulfonique en C₁₁-C₁₄ éventuellement hydroxylé substitué par un ou deux groupes alkyle en C₁-C₄, d'un acide alcanoïque en C₃ à C₆ et de tout mélange desdits sels.

11. Composition selon la revendication 10, dans laquelle l'ingrédient facilitant la solubilisation est choisi dans le groupe constitué du sel de sodium de l'acide pyrrolidone-carboxylique, du benzoate de sodium, du L-lactate de sodium, du L-ascorbate de calcium, du bicarbonate de sodium, du succinate de disodium, et de tout mélange desdits sels.

12. Composition selon l'une quelconque des revendications 1 à 11, dans laquelle l'ingrédient facilitant la solubilisation représente 0,1 à 5% p/p de la microémulsion.

13. Composition selon l'une quelconque des revendications 1 à 12, dans laquelle ladite microémulsion a une tension superficielle, à 25°C, comprise entre 22 et 30 mN/m.

14. Article de consommation comprenant une composition, telle que définie dans l'une quelconque des revendications 1 à 13, conjointement avec un contenant et éventuellement un moyen de produire un aérosol.

15. Article de consommation selon la revendication 14, sous la forme d'un désodorisant d'atmosphère ou d'une préparation capillaire ou une préparation pour la peau.

16. Article de consommation comprenant une composition, telle que définie dans l'une quelconque des revendications 1 à 13, et une base pour article de consommation.

17. Procédé pour conférer, intensifier, améliorer ou modifier les propriétés odorantes d'un produit ou d'un article, lequel procédé comprend l'ajout audit produit ou article d'une quantité efficace sur le plan olfactif d'une composition selon la revendication 1.

18. Milieu de solubilisation sans COV et sans huile comprenant:
I) de 1,0 à 45% p/p d'un système tensioactif, tel que défini dans la revendication 1;
II) de 2 à 15% p/p d'un agent facilitant la solubilisation, tel que défini dans la revendication 1; et
III) de l'eau jusqu'à 100%;
ledit milieu sans huile ayant une tension superficielle, à 25°C, comprise entre 20 et 35 mN/m.

19. Milieu de solubilisation selon la revendication 18, comprenant de 3,5 à 15% p/p d'un système tensioactif, tel que défini dans la revendication 1, et de 0,1 à 10% p/p d'un agent facilitant la solubilisation, tel que défini dans la revendication 1.

20. Milieu de solubilisation selon la revendication 18, dans lequel l'ingrédient facilitant la solubilisation est choisi dans le groupe constitué du sel de sodium de l'acide pyrrolidone-carboxylique, du benzoate de sodium, du L-lactate de sodium, du L-ascorbate de calcium, du bicarbonate de sodium, du succinate de disodium, et de tout mélange desdits sels.

21. Procédé de préparation d'une composition selon la revendication 1, dans lequel on ajoute à un milieu de solubilisation selon la revendication 18 une quantité appropriée d'huile, de préférence une huile parfumée, pour former une microémulsion transparente.
